# EUROPEAN PATENT APPLICATION

(11) **EP 2 612 919 A1**
(43) Date of publication of application: **10.07.2013**
(21) Application number: 11821878.3
(22) Date of filing: 31.08.2011
(51) Int. Cl.: C12P 19/04, A61K 31/737, A61P 19/00, A61P 43/00, C08B 37/00, C12Q 1/04, G01N 33/53, G01N 33/566

(54) **SULFATED POLYSACCHARIDE CAPABLE OF BINDING TO GROWTH FACTOR AND USE THEREOF**

(30) Priority: 29.03.2011 JP 2011071661; 02.09.2010 JP 2010196583
(71) Applicant: Sumitomo Bakelite Company Limited, Shinagawa-ku Tokyo 140-0002 (JP); Tabata, Yasuhiko, Uji-shi, Kyoto 611-0024 (JP)
(72) Inventor: TABATA Yasuhiko, Uji-shi Kyoto 611-0024 (JP); FUKUNISHI Yoshiaki, Tokyo 140-0002 (JP)
(74) Representative: Schnappauf, Georg
(86) International application number: PCT/JP2011/069796
(87) International publication number: WO 2012/029863

(57) **Abstract**

The present invention provides: a biological substance which can enhance the proliferation and differentiation of cells; a method for evaluating the proliferation and differentiation of cells, which targets the biological substance; a composition for detecting the proliferation and differentiation of cells, which contains a molecule capable of binding to the biological substance as an active ingredient; and a composition for enhancing the proliferation and differentiation of cells, which contains the biological substance as an active ingredient. According to the present invention, it is found that a part of a sulfated polysaccharide secreted from cells binds to a growth factor during the course of the proliferation and differentiation of the cells, that the amount of the sulfated polysaccharide capable of the above-mentioned binding and secreted from the cells correlates strongly with the progression of the proliferation and differentiation of the cells, and that the proliferation and differentiation of cells can be evaluated and the proliferation and differentiation of cells can be enhanced utilizing the sulfated polysaccharide.

## Description

### TECHNICAL FIELD

The present invention relates to a sulfated polysaccharide capable of binding to growth factor that is secreted from cells during the course of cell proliferation and differentiation, a method for evaluating cell proliferation and differentiation that targets the sulfated polysaccharide, a composition for detecting cell proliferation and differentiation having a molecule that binds to the sulfated polysaccharide as an active ingredient thereof, and a composition for promoting cell proliferation and differentiation having the sulfated polysaccharide as an active ingredient thereof.
The present application claims priority on the basis of Japanese Patent Application No. 2010-196583, which was filed in Japan on September 2, 2010, and Japanese Patent Application No. 2011-071661, which was filed in Japan on March 29, 2011, the contents of which are incorporated herein by reference.

### BACKGROUND ART

Stem cell transplantation is typically carried out in the field of regenerative medicine as a treatment method that has been researched and developed and partially applied practically. Although embryonic stem cells, mesenchymal stem cells harvested from bone marrow or fat tissue and the like, hematopoietic stem cells and various types of tissue stem cells (such as hepatic stem cells or neural stem cells) are used as stem cells for cell transplantation, mesenchymal stem cells can be acquired and cultured comparatively easily. Consequently, research on cell transplantation has progressed the greatest in the field of biological tissue regeneration using mesenchymal stem cells.

Examples of tissues targeted for regenerative medicine using mesenchymal stem cells include bone, cartilage, fat, muscle, tendon and nerves. In the case of bone regeneration, for example, a method is used that consists of disseminating and proliferating mesenchymal stem cells that have been cultured and proliferated in vitro in a three-dimensional scaffold, followed by transplantation and treatment, and there are cases in which this treatment has been applied (see, for example, Patent Document 1).

Normally, a polysaccharide proliferation factor must be continued to be added to the culture fluid in order to culture mesenchymal stem cells in vitro. Examples of approaches that have been developed to accomplish this include a method consisting of synthesizing proliferation factor in cells and continuously supplying the proliferation factor autocrinically, and a method that enables favorable bone and cartilage regeneration by continuously expressing transcription factor activity using a viral vector incorporating a transcription factor gene (see, for example, Patent Document 2).
However, since the body inherently possesses its own natural healing power, treatment is actually provided using that natural healing power in the case of simple fractures and the like. On the other hand, although there are cases, such as in the case of large bone defects, for which the body is unable to adequately demonstrate its inherent natural healing power, by enhancing that natural healing power by administering substances that promote cell proliferation and differentiation, for example, it is possible to realize regeneration and repair of body tissue without having to undergo stem cell transplantation or gene therapy. In addition, in the case a substance having such action is a substance that is present in the body, it is also thought to be possible to evaluate the status of cell proliferation and differentiation by using that substance as a target.

On the basis of such viewpoints, a substance present in the body is sought that is able to promote cell proliferation and differentiation and enhance the natural healing power inherently possessed by the body.

### Prior Art Documents

### Patent Documents

Patent Document 1: Japanese Unexamined Patent Application, First Publication No. 2008-29317
Patent Document 2: International Publication No. WO 2005/035014

### DISCLOSURE OF THE INVENTION

### Problems to be Solved by the Invention

With the foregoing in view, an object of the present invention is to provide a biological substance that is capable of promoting cell proliferation and differentiation. Another object of the present invention is to provide a method for evaluating cell proliferation and differentiation that uses the biological substance as a target, a composition for detecting cell proliferation and differentiation that has a molecule that binds to the biological substance as an active ingredient thereof, and a composition for promoting cell proliferation and differentiation that has the biological substance as an active ingredient thereof.

### Means for Solving the Problems

As a result of conductive extensive studies to achieve the aforementioned objects, the inventors of the present invention found that a portion of a sulfated polysaccharide secreted from cells binds to growth factor during the course of cell proliferation and differentiation, and that the amount of the sulfated polysaccharide secreted from cells that exhibits this binding activity demonstrates a strong correlation with the degree of progression of cell proliferation and differentiation. Moreover, the inventors of the present invention found that cell proliferation and differentiation can be evaluated and cell proliferation and differentiation can be promoted by using this sulfated polysaccharide that binds to growth factor, thereby leading to completion of the present invention.

More specifically, the present invention provides the inventions indicated below.

(1) A sulfated polysaccharide secreted from cells during the course of cell proliferation and differentiation that binds to growth factor.

(2) The sulfated polysaccharide described in (1), wherein the course of cell proliferation and differentiation is the course of proliferation and differentiation of osteocytes or chondrocytes.

(3) The sulfated polysaccharide described in (1) or (2), wherein the growth factor is a molecule selected from the group consisting of BMP-2 and bFGF.

(4) A method for evaluating cell proliferation and differentiation, comprising detecting the sulfated polysaccharide described in any of (1) to (3).

(5) A composition for detecting cell proliferation and differentiation having as an active ingredient thereof a molecule that binds to the sulfated polysaccharide described in any of (1) to (3).

(6) A composition for promoting cell proliferation and differentiation having as an active ingredient thereof the sulfated polysaccharide described in any of (1) to (3).

### Effects of the Invention

Since the sulfated polysaccharide discovered by the inventors of the present invention that binds to growth factor is able to stabilize or enhance the activity of growth factor, the use of this sulfated polysaccharide makes it possible to promote cell proliferation and differentiation that uses the body's natural healing power in vivo, as well as promote cell proliferation and differentiation in vitro. In addition, the degree of progression of cell proliferation and differentiation in vivo and the degree of progression of cell proliferation and differentiation in vitro can be evaluated by targeting the sulfated polysaccharide that binds to growth factor discovered by the inventors of the present invention.

### BRIEF DESCRIPTION OF THE DRAWINGS

FIG. 1 is a graph indicating the results of measuring the level of alkaline phosphatase (ALP) activity of osteoprogenitor cells cultured in bone differentiation medium (Example 1) and osteoprogenitor cells cultured in ordinary medium (Comparative Example 1).
FIG. 2 is a graph indicating the results of measuring the amount of calcium deposited by osteoprogenitor cells cultured in bone differentiation medium (Example 1) and osteoprogenitor cells cultured in ordinary medium (Comparative Example 1).
FIG. 3 is a graph indicating the results of measuring the amount of sulfated polysaccharide secreted from osteoprogenitor cells cultured in bone differentiation medium (Example 1) and osteoprogenitor cells cultured in ordinary medium (Comparative Example 1).
FIG. 4 is a graph indicating the results of measuring the amount of sulfated polysaccharide capable of binding to bFGF secreted from osteoprogenitor cells cultured in bone differentiation medium (Example 1) and osteoprogenitor cells cultured in ordinary medium (Comparative Example 1).
FIG. 5 is a graph indicating the results of measuring the amount of sulfated polysaccharide capable of binding to BMP-2 secreted from osteoprogenitor cells cultured in bone differentiation medium (Example 1) and osteoprogenitor cells cultured in ordinary medium (Comparative Example 1).
FIG. 6 is a graph indicating the results of analyzing the structures of sulfated polysaccharides secreted from osteoprogenitor cells cultured in bone differentiation medium (Example 1) and osteoprogenitor cells cultured in ordinary medium (Comparative Example 1).
FIG. 7 depicts graphs indicating the results of measuring the levels of alkaline phosphatase (ALP) activity of mesenchymal stem cells cultured in bone differentiation medium (Examples 3 to 6) and mesenchymal stem cells cultured in ordinary medium (Comparative Examples).
FIG. 8 depicts graphs indicating the results of measuring the amounts of calcium deposited by mesenchymal stem cells cultured in bone differentiation medium (Examples 3 to 6) and mesenchymal stem cells cultured in ordinary medium (Comparative Examples).
FIG. 9 depicts graphs indicating the results of measuring the amounts of sulfated polysaccharide excreted from mesenchymal stem cells cultured in bone differentiation medium (Examples 3 to 6) and mesenchymal stem cells cultured in ordinary medium (Comparative Examples).
FIG. 10 is a graph indicating the results of measuring the amount of sulfated GAG produced in a cell extract during cartilage differentiation (Example 2).
FIG. 11 is a graph indicating the results of measuring the amount of sulfated polysaccharide in culture supernatant during cartilage differentiation (Example 2).
FIG. 12 is a graph indicating the results of measuring the amount of sulfated polysaccharide capable of binding to BMP-2 in culture supernatant during cartilage differentiation (Example 2).
FIG. 13 is a graph indicating the results of analyzing the structure of sulfated polysaccharides secreted from mesenchymal stem cells cultured in cartilage differentiation medium (Example 2) and chondrocytes cultured in ordinary medium (Comparative Example 2).

### BEST MODE FOR CARRYING OUT THE INVENTION

The present invention provides a sulfated polysaccharide secreted from cells during the course of cell proliferation and differentiation that binds to growth factor. Moreover, the present invention provides a method for evaluating cell proliferation and differentiation comprising detection of that sulfated polysaccharide.

Examples of the "course of cell proliferation and differentiation" in the present invention include, but are not limited to, the course of proliferation and differentiation of osteocytes, chondrocytes, myocytes, adipocytes, hepatocytes, neurons and hemocytes. Culture systems that cause various types of stem cells to differentiate into cells of various tissues can be used for culture systems for proliferation and differentiation of these cells. Examples of stem cells include, but are not limited to, embryonic stem cells, mesenchymal stem cells harvested from bone marrow or fat tissue and the like, hematopoietic stem cells, and various types of tissue stem cells (such as hepatic stem cells or neural stem cells). In addition, examples of biological sources of cells for inducing proliferation and differentiation include mice, rats, hamsters, humans, monkeys, pigs, cows, horses, sheep, goats, chickens, wild ducks, ostriches, domestic ducks, dogs, cats and rabbits.

A method commonly known among persons with ordinary skill in the art can be used to proliferate and differentiate cells. An example of a method used to proliferate and differentiate osteocytes consists of disseminating cells able to differentiate into osteocytes, and culturing for 2 to 4 days in an ordinary medium such as medium to which 10% to 15% of fetal bovine serum has been added to a minimum essential medium such as alpha-Modified Eagle Minimum Essential Medium (α-MEM) or Dulbecco's Modified Eagle Minimum Essential Medium (DMEM). Here, examples of cells able to differentiate into osteocytes include osteoblasts, osteoprogenitor cells, mesenchymal cells (including mesenchymal stem cells) and cell lines thereof (such as MC3T3-E1 or CH310T1/2). Subsequently, the medium is replaced with differentiation medium such as a medium obtained by adding β-glycerophosphate, ascorbic acid (or a derivative thereof in the form of ascorbyl-2-phosphate) and dexamethasone to the aforementioned ordinary medium followed by culturing for 21 days to 28 days. During culturing in the differentiation medium, for example, conditions consisting of culturing in gaseous 5% CO₂ at 37°C can be applied while replacing the medium every 3 to 4 days. One or more types of factors known to induce differentiation of mesenchymal stem cells to osteoblasts (such as BMP-2, BMP-3, BMP-4, BMP-7, bFGF or TGF-β1) can also be added to the differentiation medium.

Differentiation to osteocytes can be detected with, for example, alkaline phosphatase activity. In addition, this differentiation can also be detected by targeting calcium deposition. A commercially available kit, such as an alkaline phosphatase assay kit (LabAssay™ ALP, Wako Pure Chemical Industries, Ltd.) or a calcium assay kit (Wako Calcium C-Test, Wako Pure Chemical Industries, Ltd.), can be used to detect these parameters.

A method described in the literature (such as Alastair M. Mackay et al., Tissue Engineering Vol. 4 (1998), pp. 415-428) can be used to proliferate and differentiate chondrocytes. In this method, after separating cultured human mesenchymal stem cells with trypsin and dispersing into individual cells in a centrifuge tube, the cells are dispersed in differentiation medium obtained by adding a reagent (consisting of 10 µg/mL TGF-β3, 100 nM dexamethasone, 50 µg/mL ascorbic acid, 100 µg/mL sodium pyruvate, 40 µg/mL proline, 6.25 µg/mL bovine insulin, 6.25 µg/mL transferrin, 5.33 µg/mL linoleic acid and 1.25 mg/mL bovine serum albumin) to Dulbecco's modified Eagle medium (DMEM), followed by producing a cell pellet by centrifuging the cells, and then culturing for 14 days as is to cause the cells to differentiate into cartilage. Confirmation of differentiation into cartilage can be made by staining each type of extracellular matrix with antibody.

A method described in the literature (such as Keiichi Fukuda, Artificial Organs Vol. 25 (2001), pp. 187-193) can be used to proliferate and differentiate cardiac myocytes. In this method, human mesenchymal cells are differentiated into cardiac muscle by culturing for 24 hours or more in differentiation medium obtained by adding 3 µM 5-azacytidine to Iscove's modified DMEM containing 20% fetal bovine serum. Differentiation into cardiac muscle can be confirmed by microscopically observing spontaneous pulsation of the cells.

A method described in the literature (such as Eun Ji Gang et al., Stem Cells 22 (2004), pp. 617-624) can be used to proliferate and differentiate skeletal myocytes. In this method, human mesenchymal stem cells are differentiated into skeletal muscle by culturing for 6 weeks in differentiation medium obtained by adding a reagent (consisting of 5% horse serum, 0.1 µM dexamethasone and 50 µM hydrocortisone) to DMEM containing 15% fetal bovine serum. Differentiation into skeletal muscle can be detected by fluorescent staining of MyoD1 characteristic to skeletal myocytes.

A method described in the literature (such as Kentaro Nagane et al., Tissue Engineering: Part A Vol. 16 (2010), pp. 21-31) can be used to proliferate and differentiate adipocytes. In this method, rat mesenchymal stem cells are differentiated into adipocytes by culturing for 6 days in a differentiation medium obtained by adding a reagent (consisting of 450 µM 3-isobutyl-1-methylxanthine, 50 µg/mL insulin, 60 µM indometacin and 10 µM troglitazone) to α-MEM containing 10% calf serum. Differentiation into adipocytes can be detected by staining with Oil Red or measuring GPDH enzyme activity.

A method described in the literature (such as Agnieszka Banas et al., Hepatology July (2007), pp. 219-228) can be used to proliferate and differentiate hepatocytes. In this method, human mesenchymal stem cells are differentiated into hepatic parenchymal cells by culturing for 3 weeks in a differentiation medium obtained by adding a reagent (consisting of 300 ng/mL FGF1, 25 ng/mL FGF4 and 150 ng/mL HGF) to DMEM containing 10% fetal bovine serum, and further culturing for 2 weeks in a medium obtained by adding a reagent (consisting of 30 ng/mL oncostatin M and 2 × 10⁻⁵ M dexamethasone) to the differentiation medium. Differentiation into hepatic parenchymal cells can be detected by measuring albumin synthesis ability or ammonium metabolism of the cells.

A method described in the literature (such as Ju Ah Jeong, et al., NeuroReport Vol. 15 (2004), pp. 1731-1734) can be used to proliferate and differentiate nerve cells. In this method, human mesenchymal stem cells are differentiated into nerve cells by culturing for 24 hours in a medium obtained by adding a reagent (consisting of 25 mM potassium chloride, 2% dimethylsulfoxide (DMSO), 100 µM butylated hydroxyanisole, 5µg/mL insulin, 100 µg/mL transferrin, 20 mM progesterone, 100 µM putrescine, 30 nM sodium selenite, 20 ng/mL bFGF and 10 mM Forskolin) to DMEM containing 20% fetal bovine serum. Differentiation into nerve cells can be detected by measuring the expression of a group of genes characteristic to nerve cells.

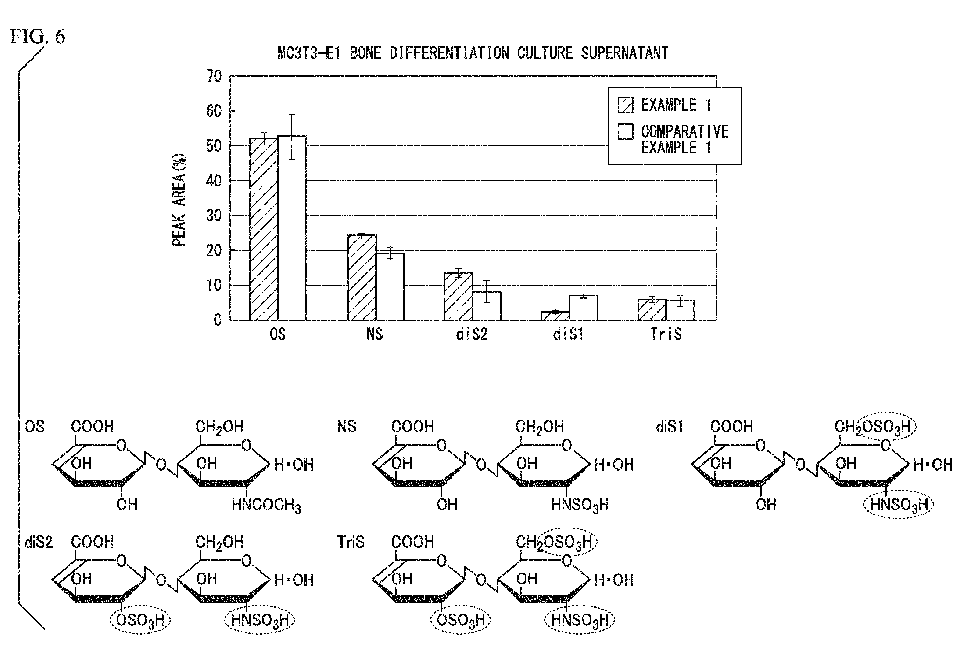

In addition, in the present invention, a "sulfated polysaccharide" refers to a polysaccharide having a sulfate group in a molecule thereof. There are no particular restrictions on the sulfated polysaccharide in the present invention provided it is secreted during the course of cell proliferation and differentiation and is able to bind to growth factor. Preferably, it is a sulfated polysaccharide that contains NS and diS2 structures as shown in FIG. 6 at a higher ratio than control values and contains a diS1 structure as shown in FIG. 6 at a lower ratio than a control value. Values of the ratios of NS, diS2 and diS1 structures in a sulfated polysaccharide secreted by cells not undergoing cell proliferation and differentiation (such as values of the ratios of NS, diS2 and diS1 structures in the sulfated polysaccharide in the case of culturing cells able to differentiate into osteocytes in an ordinary medium that does not induce differentiation in the case of detecting proliferation and differentiation of osteocytes) can be used for the control values.

In addition, in the present invention, "growth factor" refers to an endogenous protein that promotes proliferation and differentiation of specific cells. Examples of "growth factors" bound by the sulfated polysaccharide of the present invention include, but are not limited to, BMP-2 and bFGF.

The sulfated polysaccharide that binds to growth factor can be prepared by contacting a cell culture supernatant with a carrier (such as heparin-Sepharose) having a growth factor or fragment thereof bound thereon, and then recovering the sulfated polysaccharide that binds to the growth factor on the carrier.

Detection of sulfated polysaccharide can be carried out with, for example, a 1,9-dimethylmethylene blue (DMB) assay. Detection of sulfated polysaccharide can also be carried out using a molecule that specifically binds to the sulfated polysaccharide (such as an antibody that binds to the sulfated polysaccharide or a peptide of that binding site, or a growth factor that binds to the sulfated polysaccharide or a peptide of that binding site).

Antibody can be acquired by immunizing an immune animal with antigen (sulfated polysaccharide that binds to growth factor) and purifying from the resulting antiserum by conventional means (such as salting out, centrifugal separation, dialysis or column chromatography) in the case of polyclonal antibody. In addition, monoclonal antibody can be produced according to a hybridoma method (described in, for example, Kohler & Milstein, Nature, 256:495(1975)) or recombinant DNA method (described in, for example, P.J. Delves, Antibody Production: Essential Techniques, 1997, Wiley; P. Shepherd and C. Dean, Monoclonal Antibodies, 2000 Oxford University Press; and Vandamme A.M. et al., Eur. J. Biochem. 192:767-775(1990)). These antibodies may be labeled as necessary. Radioactive substances, fluorescent pigments, chemoluminescent substances, enzymes or coenzymes can be used for the label, and specific examples thereof include radioisotopes, fluorescein, rhodamine, dansyl chloride, luciferase, peroxidase, alkaline phosphatase, lysozyme and biotin/avidin. When formulating the antibody in the form of a composition such as a diagnostic agent, the antibody can be obtained in an arbitrary preparation form by employing arbitrary means in accordance with the objective thereof. For example, a purified antibody can be prepared by measuring the antibody titer and adding a suitable carrier (to be subsequently described).

A growth factor or a peptide of that binding site can be prepared by incorporating DNA encoding the same into a suitable expression vector, recovering a transformant obtained by introducing this into suitable host cells, and obtaining an extract of the transformant, followed by purifying by applying to ion exchange, reverse phase or gel filtration chromatography, or to affinity chromatography in which a sulfated polysaccharide or antibody bound to the growth factor is immobilized on a column, or by combining a plurality of these purification means. The growth factor or peptide of that binding site may also be labeled as necessary in the same manner as in the case of the aforementioned antibody.

The present invention also provides a composition for detecting cell proliferation and differentiation that has as an active ingredient thereof a molecule that binds to this type of sulfated polysaccharide.

As a result of having detected a sulfated polysaccharide using the composition of the present invention, the process of cell proliferation and differentiation can be evaluated as proceeding in the case the sulfated polysaccharide is detected in an amount that is greater than a control value. For example, the value of the secreted amount of the sulfated polysaccharide in cells in which cell proliferation and differentiation is not proceeding (such as the value of the secreted amount of the sulfated polysaccharide in the case of culturing cells able to differentiate into osteocytes in ordinary medium that does not induce differentiation in the case of detecting proliferation and differentiation of osteocytes) can be used as a control value. In addition, the process of cell proliferation and differentiation can be evaluated as proceeding in the case the NS and diS2 structures shown in FIG. 6 are detected at a higher ratio than a control value, or in the case the diS1 structure shown in FIG. 6 is detected at a lower ratio than a control value, in the secreted sulfated polysaccharide. For example, values of the ratios of NS, diS2 and diS1 structures in a sulfated polysaccharide secreted by cells not undergoing cell proliferation and differentiation (such as values of the ratios of NS, diS2 and diS1 structures in the sulfated polysaccharide in the case of culturing cells able to differentiate into osteocytes in an ordinary medium that does not induce differentiation in the case of detecting proliferation and differentiation of osteocytes) can be used for the control values. This composition of the present invention can be used as an experimental reagent for evaluating the degree of cell proliferation and differentiation in an experiment using culture cells, or for evaluating the degree of cell proliferation and differentiation in an animal experiment. In addition, the composition of the present invention can be used as a diagnostic drug for evaluating the degree of cell proliferation and differentiation for the purpose of accurately diagnosing the progression of treatment with respect to regenerative medicine in a human body, for example.

The present invention also provides a composition for promoting osteocyte proliferation and differentiation that has as an active ingredient thereof the sulfated polysaccharide that binds to growth factor. This composition of the present invention can be in the form of a reagent used in pharmaceutical compositions, foods and beverages (including animal feed) or for research and development purposes (such as in vitro and in vivo experiments or the production of artificial tissues and organs such as artificial bone).

The composition of the present invention can be preferably used as a pharmaceutical composition administered to prevent or treat diseases caused by damage to cells, tissues or organs or by abnormal cell proliferation and differentiation, or as a food or beverage consumed on a regular basis, in order to have an action that promotes cell proliferation and differentiation by stabilizing or enhancing growth factor activity.

The composition of the present invention can be formulated according to commonly known pharmaceutical techniques. For example, the composition of the present invention can be used orally or parenterally in the form of a capsule, tablet, pill, liquid, powder, granules, grains, film-coated preparation, pellet, lozenge, sublingual preparation, chewable preparation, buccal preparation, paste, syrup, suspension, elixir, emulsion, liniment, ointment, plaster, poultice, transcutaneously absorbed preparation, lotion, inhalant, aerosol, injection or suppository.

In a preparation of the composition of the present invention, the composition of the present invention can be suitably combined with a pharmaceutically acceptable carrier or carrier permitted to be used in a food or beverage, specific examples of which include sterile water or physiological saline, vegetable oils, solvents, bases, emulsifiers, suspending agents, surfactants, stabilizers, flavoring agents, fragrances, excipients, vehicles, preservatives, binders, diluents, tonicity agents, analgesics, extenders, disintegration agents, buffers, coating agents, lubricants, colorants, sweeteners, thickeners, correctives, solubilizing agents, and other additives.

In the case of using the composition of the present invention as a pharmaceutical composition, it may be used in combination with a known pharmaceutical composition used for the treatment and prevention of diseases caused by damage to cells, tissues or organs or by abnormal cell proliferation and differentiation.

In the case of using the composition of the present invention as a food or beverage, the food or beverage can be a health food, functional food, food for specified health uses, nutritional supplement, medical food, food additives or animal feed. A food or beverage of the present invention can be ingested as a composition as previously described, or can be ingested in the form of various foods and beverages. Specific examples of foods and beverages include products containing oil such as edible oils, salad dressing, mayonnaise or margarine; liquid foods such as a soup, milk drinks, soft drinks, tea drinks, alcoholic beverages, drinks, jellied beverages or functional beverages; carbohydrate-containing foods such as rice, noodles or bread: processed animal foods such as ham or sausage; processed marine products such as pressed fish, dried fish or salted squid; processed vegetable foods such as pickles; semi-solid foods such as jelly or yogurt; fermented foods such as fermented beverages; various types of confections such as Western confections, Japanese confections, candy, gum, gummi drops, chilled confections or frozen confections; boil-in-the-bag foods such as curry sauce, sweet bean sauce or Chinese style soup; and instant foods and microwaved foods such as instant soup or instant miso soup. Moreover, additional examples include health foods and beverages prepared in the form of powders, granules, tablets, capsules, liquids, pastes or jelly. Although the composition of the present invention can be used for animals, including humans, it can be used in animals other than humans such as various species of livestock, poultry, pets or experimental animals. Specific examples include, but are not limited to, pigs, cows, horses, sheep, goats, chickens, wild ducks, ostriches, domestic ducks, dogs, cats, rabbits, hamsters, mice, rats and monkeys.

A food or beverage of the present invention can be produced using known production technology employed in the relevant field. One type or two or more types of active ingredients may be added to the food or beverage in order to promote cell proliferation and differentiation. In addition, the food or beverage may also be in the form of a functional food or beverage by combining with other components or other functional foods that demonstrate a function other than the promotion of cell proliferation and differentiation.

In the case of administering or ingesting the composition of the present invention, the dosage or ingested amount thereof is suitably selected corresponding to the age, body weight, symptoms or health status of a subject or the type of composition (such as a pharmaceutical, food or beverage). For example, the dosage or ingested amount of the composition of the present invention per administration is typically 0.01 mg/kg of body weight to 100 mg/kg of body weight. In this manner, the present invention also provides a method for promoting cell proliferation and differentiation in a subject by administration of the composition of the present invention to the subject or ingestion of the composition of the present invention by the subject. In addition, the present invention also provides a method for preventing or treating a disease caused by damage to cells, tissues or organs or caused by abnormal cell proliferation and differentiation in a subject by administering the composition of the present invention to the subject.

A product of the composition of the present invention (pharmaceutical, food, beverage or reagent) or the instructions thereof may be provided with an indication to the effect that the product is used to promote cell proliferation and differentiation. Here, "providing an indication on a product or instructions" refers to the providing of an indication on the body, container or package of a product, or providing an indication in instructions, inserts, advertisements or other printed matter that discloses information about the product. In an indication to the effect that the composition of the present invention is used to promote cell proliferation of differentiation, information can be included regarding the mechanism by which cell proliferation and differentiation are promoted as a result of administering or ingesting the composition of the present invention. In addition, in an indication to the effect that the composition of the present invention is used to promote cell proliferation and differentiation, information can be included regarding the use thereof for the prevention or treatment of diseases caused by damage to cells, tissues or organs or by abnormal cell proliferation and differentiation.

### Examples

Although the following provides a more detailed explanation of the present invention based on examples and comparative examples, the present invention is not limited to the following examples.

### [Example 1] Culturing of Mouse-Derived Osteoprogenitor Cells in Differentiation Medium

MC3T3-E1 mouse-derived osteoprogenitor cells were respectively disseminated in three 10 cm culture dishes (Corning Inc.) containing medium obtained by adding 10% fetal bovine serum to α-MEM to 3.0 × 10⁵ cells per culture dish followed by culturing for 2 days at 37°C in a 5% CO₂ incubator. 50 µg/mL ascorbic acid (Nacalai Tesque Inc.), 10 mM β-glycerophosphate (Sigma Corp.) 10⁻⁸ M dexamethasone (Nacalai Tesque Inc.) and 100 ng/ml rhBMP-2 were added to the medium to initiate induction of differentiation. The medium was replaced every 3 to 4 days, and the cells and supernatant were sampled after culturing for 1 week at 37°C in a 5% CO₂ incubator.

### [Comparative Example 1]

MC3T3-E1 mouse-derived osteoprogenitor cells were respectively disseminated in three 10 cm culture dishes (Corning Inc.) containing medium obtained by adding 10% fetal bovine serum to α-MEM to 3.0 × 10⁵ cells per culture dish followed by culturing for 2 days at 37°C in a 5% CO₂ incubator. The same medium was replaced every 3 to 4 days, and the cells and supernatant were sampled after culturing for 1 week at 37°C in a 5% CO₂ incubator.

### [Evaluation Methods]

### (1) Measurement of Alkaline Phosphatase (ALP) Activity

### (Indicator of Osteocyte Differentiation)

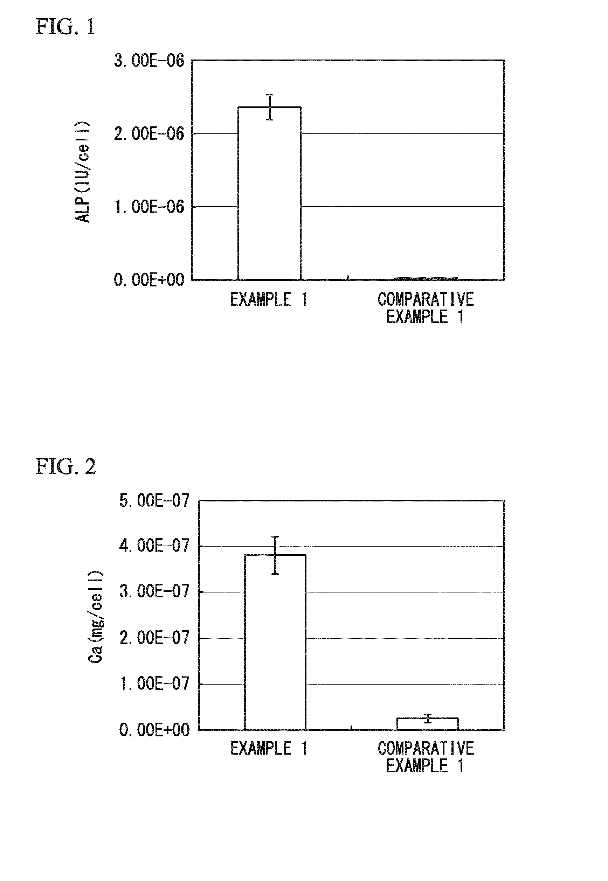

The sampled cells were lysed in a solution (containing 3 M sodium chloride (Wako Pure Chemical Industries, Ltd.), 0.3 M trisodium citrate (Nacalai Tesque Inc.) and 200 mg/L sodium dodecyl sulfate (Nacalai Tesque Inc.) followed by measurement of the level of ALP activity using an alkaline phosphatase assay kit (LabAssay™ ALP, Wako Pure Chemical Industries, Ltd.). The value obtained by measuring absorbance at 405 nm with a microplate reader (VersaMax, Molecular Devices Corp.) was divided by the number of cells to determine the level of ALP activity per cell (FIG. 1).

### (2) Measurement of Amount of Calcium Deposition

### (Indicator of Osteocyte Differentiation)

The sampled cells were lysed in a solution (containing 3 M sodium chloride (Wako Pure Chemical Industries, Ltd.), 0.3 M trisodium citrate (Nacalai Tesque Inc.) and 200 mg/L sodium dodecyl sulfate (Nacalai Tesque Inc.) followed adding an equal amount of 1 M aqueous HCL solution (Nacalai Tesque Inc.) and allowing to stand undisturbed for 4 hours at 4°C, and measuring the amount calcium deposited using a calcium assay kit (Wako Calcium C-Test, Wako Pure Chemical Industries, Ltd.). The value obtained by measuring absorbance at 570 nm with a microplate reader (VersaMax, Molecular Devices Corp.) was divided by the number of cells to determine the amount of calcium deposited per cell (FIG. 2).

### (3) Measurement of Amount of Sulfated Polysaccharide

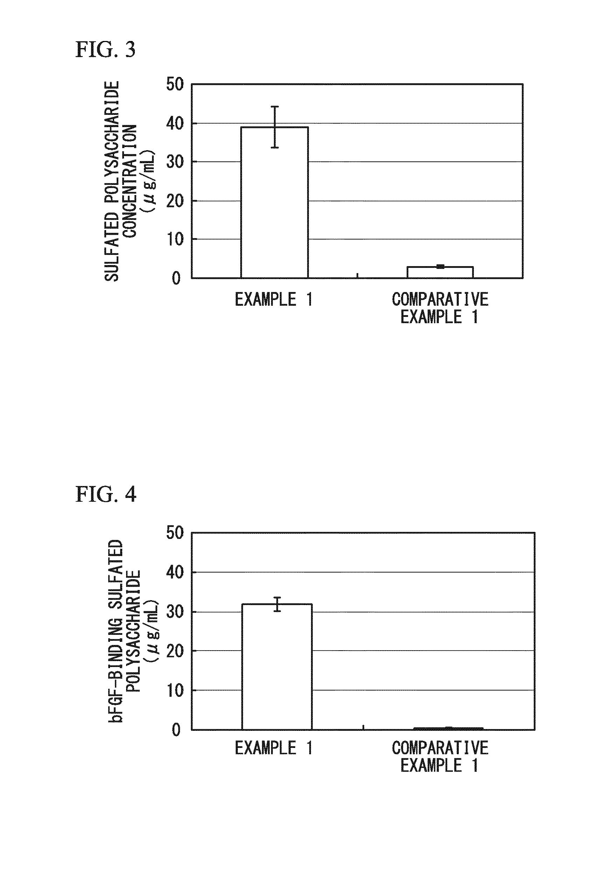

Sulfated polysaccharide present in the sampled cell supernatants were measured using a 1,9-dimethylmethylene blue (DMB) assay. The amount of sulfated polysaccharide present in 1 mL of culture supernatant was determined from a calibration curve obtained from values obtained by measuring absorbance at 525 nm with a microplate reader (VersaMax, Molecular Devices Corp.) using a solution obtained by dissolving chondroitin sulfate A sodium salt in medium containing 10% fetal bovine serum in α-MEM (FIG. 3).

### (4) Measurement of Amount of Sulfated Polysaccharide Capable of Binding to bFGF

5 µL aliquots of heparin-Sepharose (GE Healthcare Corp.) were completely dispersed and washed twice with 50 µL of 10 mM phosphate buffer followed by treating for 10 minutes by respectively adding 100 µL of a solution obtained by dissolving bFGF in 10 mM phosphate buffer and 100 µg/mL of 10 mM phosphate buffer. After removing the supernatant and washing twice with 200 µL of 10 mM phosphate buffer, the sampled culture supernatant was added followed by treating for 30 minutes and recovering the supernatant. The amount of sulfated polysaccharide present in recovered supernatant was measured using a 1, 9-dimethylmethylene blue (DMB) assay. The amount of sulfated polysaccharide present in 1 mL of culture supernatant capable of binding to bFGF was determined by determining the amount of sulfated polysaccharide remaining in the supernatant from a calibration curve obtained from values obtained by measuring absorbance at 525 nm with a microplate reader (VersaMax, Molecular Devices Corp.) using a solution obtained by dissolving chondroitin sulfate A sodium in 10 mM phosphate buffer, and subtracting from the amount of sulfated polysaccharide present in the culture supernatant (FIG. 4).

### (5) Measurement of Amount of Sulfated Polysaccharide Capable of Binding to rhBMP-2

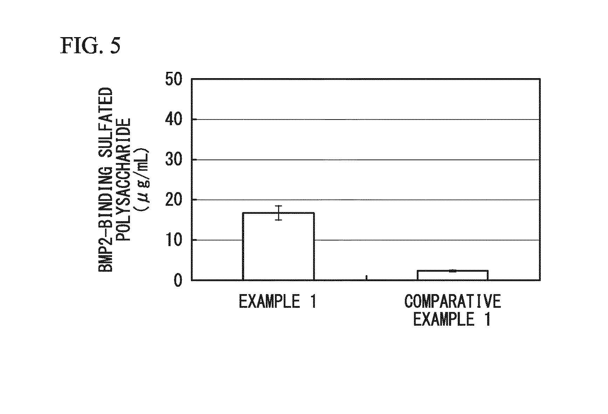

Measurement was carried out in the same manner as measurement of sulfated polysaccharide capable of binding to bFGF with the exception of using a solution obtained by dissolving rhBMP-2 in 10 mM phosphate buffer to 100 µg/mL instead of a solution obtained by dissolving bFGF in 10 mM phosphate buffer to 100 µg/mL (FIG. 5).

### (6) Confirmation of Sulfated Polysaccharide Structure

MilliQ was respectively added to 100 µL of medium obtained by adding 10% fetal bovine serum to 100 µL of the culture supernatant described in Example 1 and α-MEM; or to 250 µL of medium obtained by adding 10% fetal bovine serum to 250 µL of the culture supernatant described in Comparative Example 1 and α-MEM; and brought to a total volume of 500 µL. The resulting solutions were subjected to centrifugal separation for 30 minutes at 14000G and 25°C using 0.5 mL of the Amicon Ultra™ 10K ultrafiltration membrane (Millipore Corp.). 480 µL of MilliQ were again added followed by again centrifuging for 40 minutes at 14000G and 25°C. Subsequently, 10 µL aliquots were sampled from 25 µL aliquots of the recovered solutions, followed by the addition of 6 µL of digestion buffer (containing 10 mM sodium acetate trihydrate (Nacalai Tesque Inc.) and 10 mM potassium acetate hydrate (Nacalai Tesque Inc.), pH = 7.0), 5 µL of a solution obtained by dissolving heparinase (Seikagaku Corp.) in 0.1% aqueous BSA (Equitech-Bio Inc.) solution to a concentration of 0.5 U/mL, and 5 µL of a solution obtained by dissolving heparitinase (Seikagaku Corp.) in 0.1% aqueous BSA (Equitech-Bio Inc.) solution to a concentration of 0.5 U/mL, further adding MilliQ to a total volume of 30 µL and incubating for 1 hour at 37°C. Sugar chains were recovered by purifying 20 µL aliquots of these solutions and a 20 µL aliquot of a 100 µM mixed aqueous solution of six types of disaccharides contained in an unsaturated heparan/heparin-disaccharide kit (Seikagaku Corp.) using the BlotGlyco™ sugar chain purification kit (Sumitomo Bakelite Co., Ltd.). 5 µL aliquots of the recovered sugar chain solutions were analyzed by high-performance liquid chromatography ((LaChrom Elite, Hitachi High-Technologies Co., Ltd.). A normal phase chromatography column (TSK-GEL, Amide-80, Tosoh Corp.) was used for the column, and 50 mM aqueous ammonium formate solution (pH 4.4) and acetonitrile were used for the mobile phase. The mobile phase was given a concentration gradient so that the 50 mM aqueous ammonium formate solution (pH 4.4) changed from 20% to 41.7% over the course of 80 minutes, and sugar chain solutions were detected by fluorescence (excitation wavelength: 330 nm, fluorescence wavelength: 420 nm) by applying at a flow rate of 0.4 mL/min and at a column temperature of 30°C. The area of the medium was subtracted from the area of the culture supernatant for the peak corresponding to the elution time of a reference disaccharide peak from the resulting HPLC charts. Each of the peak areas calculated in this manner was then divided by the total area of the six peaks and multiplied by 100 to calculate peak area (%) followed by a comparison of those values.

As a result, diS1 was formed in large amounts in ordinary medium, while NS and diS2 were formed in large amounts in differentiation medium (FIG. 6).

### [Example 2]

Bone marrow aspirate collected from rat femur and tibia was disseminated in a culture flask, and after culturing at 37°C for 2 days in a 5% CO₂ incubator, the culture fluid was replaced and suspended cells were removed. The cells that adhered were further sub-cultured for 1 to 2 days at 37°C, and bone marrow-derived mesenchymal stem cells (MSC) obtained by culturing at 37°C until 70% to 90% confluent were suspended at 2.0 × 10⁶ cells/mL in medium obtained by adding 1% fetal bovine serum to DMEM (high glucose), followed by the addition of 10 ng/mL rhTGF-β3, 20 mM L-ascorbyl phosphate (Sigma Corp.), 100 nM dexamethasone (Nacalai Tesque Inc.), 40 µg/mL L-proline (Nacalai Tesque Inc.), 2mM of sodium pyruvate (Nacalai Tesque Inc.), 1 vol% ITS+ Premix (BD Biosciences Corp.) and 100 ng/mL rhBMP-2, and adding 0.5 mL aliquots to ten 15 mL centrifuge tubes to initiate induction of cartilage differentiation. After culturing at 37°C in a 5% CO₂ incubator and replacing the medium once every 3 to 4 days, the cells were sampled after 14 days and 21 days, while the culture supernatant was sampled after 4 days, 7 days, 11 days, 14 days, 18 days and 21 days.

### [Example 3]

Bone marrow aspirate collected from rat femur and tibia was disseminated in a culture flask, and after culturing at 37°C for 2 days in a 5% CO₂ incubator, the medium was replaced and suspended cells were removed. The cells were further sub-cultured for 1 to 2 days at 37°C, and mesenchymal stem cells (MSC) obtained by culturing at 37°C until 70% to 90% confluent were disseminated at 5.0 × 10⁴ cells/mL in a 6-well plate (Corning Inc.) containing medium obtained by adding 15% fetal bovine serum to α-MEM, followed by culturing for 2 days at 37°C in a 5% CO₂ incubator. Bone differentiation was initiated by adding to medium containing 50 µg/mL ascorbic acid (Nacalai Tesque Inc.), 10 mM β-glycerophosphate (Sigma Corp.) and 10⁻⁸ M dexamethasone (Nacalai Tesque Inc.). After culturing at 37°C in a 5% CO₂ incubator and replacing the medium once every 3 to 4 days, the cells were sampled after 10 days and 21 days, while the culture supernatant was sampled after 3 days, 7 days, 10 days, 14 days, 18 days and 21 days.

### [Example 4]

The procedure was carried out in the same manner as Example 3 with the exception of inducing bone differentiation by adding to the medium 50 µg/mL ascorbic acid (Nacalai Tesque Inc.), 10 mM β-glycerophosphate (Sigma Corp.), 10⁻⁸ M dexamethasone (Nacalai Tesque Inc.) and 100 ng/mL rhBMP-2.

### [Example 5]

Human mesenchymal stem cells (hMSC) (PT-2501, Lonza Inc.) were disseminated at 5.0 × 10⁴ cells/mL into 3 wells of a 6-well plate (Sumiron Co., Ltd.) containing mesenchymal stem cell serum-free medium (STK2, Two Cells Inc., Ltd.) and cultured for 3 days at 37°C in a 5% CO₂ incubator. The medium was switched to mesenchymal stem cell bone differentiation serum-free medium (STK3, Two Cells Inc., Ltd.) to initiate induction of bone differentiation. After culturing at 37°C in a 5% CO₂ incubator and replacing the medium once every 3 to 4 days, the cells and supernatant were sampled after 4 days, 7 days, 14 days and 21 days.

### [Example 6]

Immortalized human mesenchymal stem cells (hMSC) (huBM01-E6E7-hTERT clone 51, acquired from the Toguchida Laboratory) were disseminated at 5. 0 × 10⁴ cells/mL into a 6-well plate (Corning Inc.) containing medium obtained by adding 10% fetal bovine serum to DMEM (low glucose), followed by incubating for 2 days at 37°C in a 5% CO₂ incubator. Bone differentiation was initiated by adding to the medium 50 µg/mL ascorbic acid (Nacalai Tesque Inc.), 10 mM β-glycerophosphate (Sigma Corp.) and 10⁻⁸ M dexamethasone (Nacalai Tesque Inc.). After culturing at 37°C in a 5% CO₂ incubator and replacing the medium once every 3 to 4 days, the cells were sampled after 10 days and 21 days, while the supernatant was sampled after 4 days, 8 days, 11 days, 14 days, 17 days and 21 days.

### [Comparative Example 2]

Bone marrow-derived mesenchymal stem cells (MSC) obtained in the same manner as Example 2 were suspended at 2.0 × 10⁶ cells/mL in medium obtained by adding 1% fetal bovine serum to DMEM (high glucose), and 0.5 mL aliquots were added to ten 15 mL centrifuge tubes (BD Biosciences Corp.) followed by culturing at 37°C in a 5% CO₂ incubator. The medium was replaced once every 3 to 4 days, the cells were sampled after 14 days and 21 days, and the culture supernatant was sampled after 4 days, 7 days, 11 days, 14 days, 18 days and 21 days.

### [Comparative Example 3]

Mesenchymal stem cells (MSC) obtained in the same manner as Example 3 were disseminated at 5.0 × 10⁴ cells/mL in a 6-well plate (Corning Inc.) containing medium obtained by adding 15% fetal bovine serum to α-MEM, followed by culturing for 2 days at 37°C in a 5% CO₂ incubator. 50 µg/mL ascorbic acid (Nacalai Tesque Inc.) and 10 mM β-glycerophosphate (Sigma Corp.) were then added to the medium, and culturing was continued at 37°C in a 5% CO₂ incubator. The medium was replaced once every 3 to 4 days, the cells were sampled after 10 days and 21 days, and the culture supernatant was sampled after 3 days, 7 days, 10 days, 14 days, 18 days and 21 days.

### [Comparative Example 4]

The procedure was carried out in the same manner as Comparative Example 3 with the exception of adding rhBMP-2 to the medium at 100 ng/mL.

### [Comparative Example 5]

Human mesenchymal stem cells (hMSC) (PT-2501, Lonza Inc.) were disseminated at 5.0 × 10⁴ cells/mL into a 6-well plate (Sumiron Co., Ltd.) containing mesenchymal stem cell serum-free medium (STK2, Two Cells Inc., Ltd.) and cultured for 3 days at 37°C in a 5% CO₂ incubator. After continuing to culture at 37°C in a 5% CO₂ incubator using the same medium, the medium was replaced once every 3 to 4 days, and the cells and culture supernatant were sampled after 4 days, after 7 days, after 14 days and after 21 days.

### [Comparative Example 6]

Immortalized human mesenchymal stem cells (hMSC) (huBM01-E6E7-hTERT clone 51, acquired from the Toguchida Laboratory) were disseminated at 5. 0 × 10⁴ cells/mL into a 6-well plate (Corning Inc.) containing medium obtained by adding 10% fetal bovine serum to DMEM (low glucose), followed by incubating for 2 days at 37°C in a 5% CO₂ incubator. 50 µg/mL ascorbic acid (Nacalai Tesque Inc.) and 10 mM β-glycerophosphate (Sigma Corp.) were then added to the medium, and culturing was continued at 37°C in a 5% CO₂ incubator. The medium was replaced once every 3 to 4 days, the cells were sampled after 14 days and 21 days, and the culture supernatant was sampled after 4 days, 8 days, 11 days, 14 days, 17 days and 21 days.

### [Evaluation Methods]

### (1) Measurement of Alkaline Phosphatase (ALP) Activity

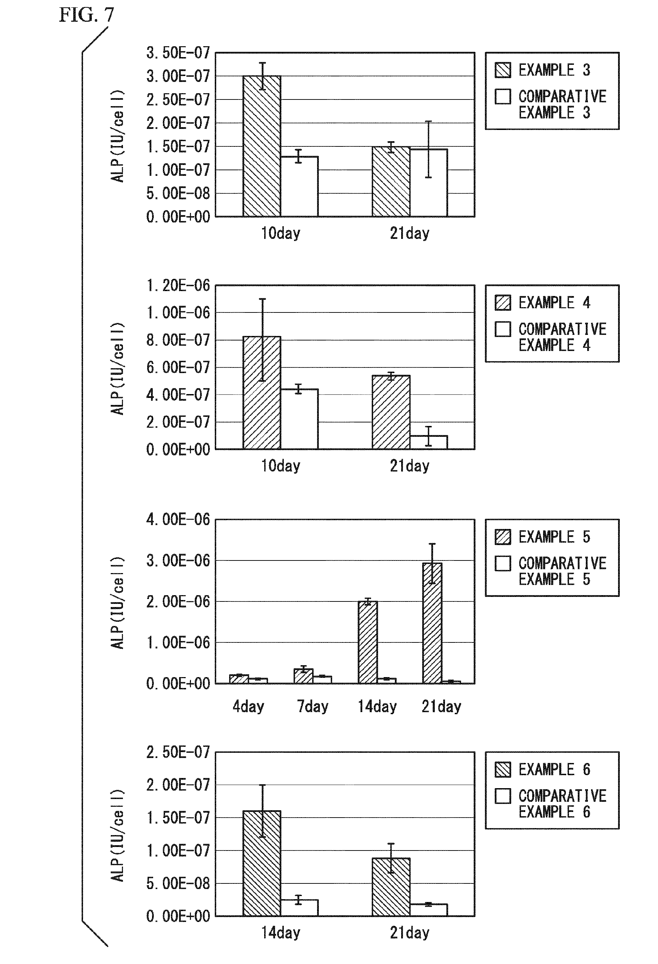

The sampled cells were lysed in a solution (containing 3 M sodium chloride (Wako Pure Chemical Industries, Ltd.), 0.3 M trisodium citrate (Nacalai Tesque Inc.) and 200 mg/L sodium dodecyl sulfate (Nacalai Tesque Inc.) followed by measurement of the level of ALP activity using an alkaline phosphatase assay kit (LabAssay™ ALP, Wako Pure Chemical Industries, Ltd.). The value obtained by measuring absorbance at 405 nm with a microplate reader (VersaMax, Molecular Devices Corp.) was divided by the number of cells to determine the level of ALP activity per cell (FIG. 7).

### (2) Measurement of Amount of Calcium Deposition

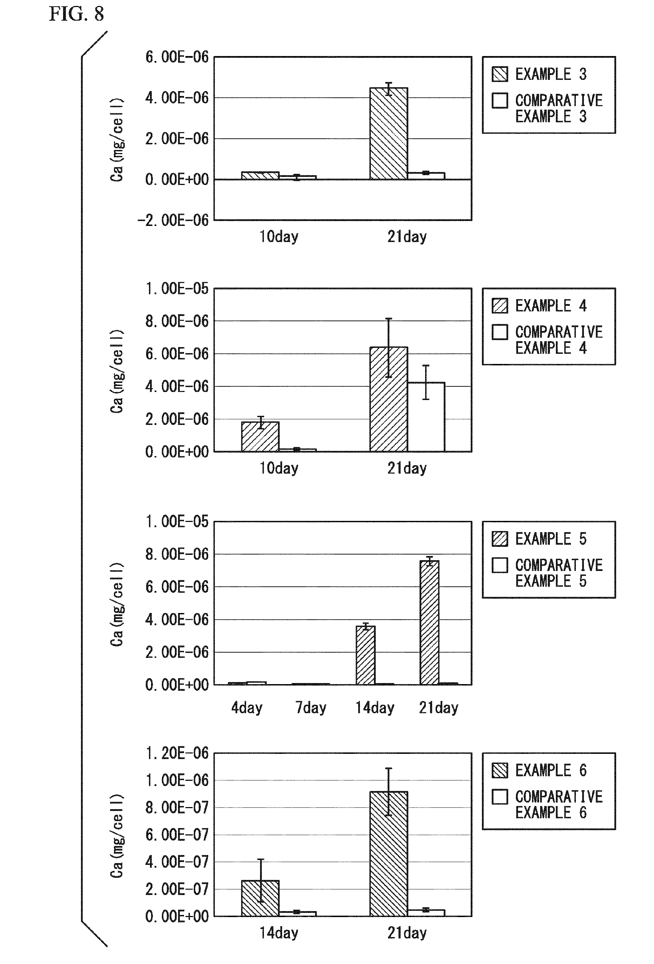

The sampled cells were lysed in a solution (containing 3 M sodium chloride (Wako Pure Chemical Industries, Ltd.), 0.3 M trisodium citrate (Nacalai Tesque Inc.) and 200 mg/L sodium dodecyl sulfate (Nacalai Tesque Inc.) followed adding an equal amount of 1 M aqueous HCL solution (Nacalai Tesque Inc.) and allowing to stand undisturbed for 4 hours at 4°C, and measuring the amount of calcium deposited using a calcium assay kit (Wako Calcium C-Test, Wako Pure Chemical Industries, Ltd.). The value obtained by measuring absorbance at 600 nm with a microplate reader (VersaMax, Molecular Devices Corp.) was divided by the number of cells to determine the amount of calcium deposited per cell (FIG. 8).

### (3) Measurement of Amount of Sulfated Polysaccharide

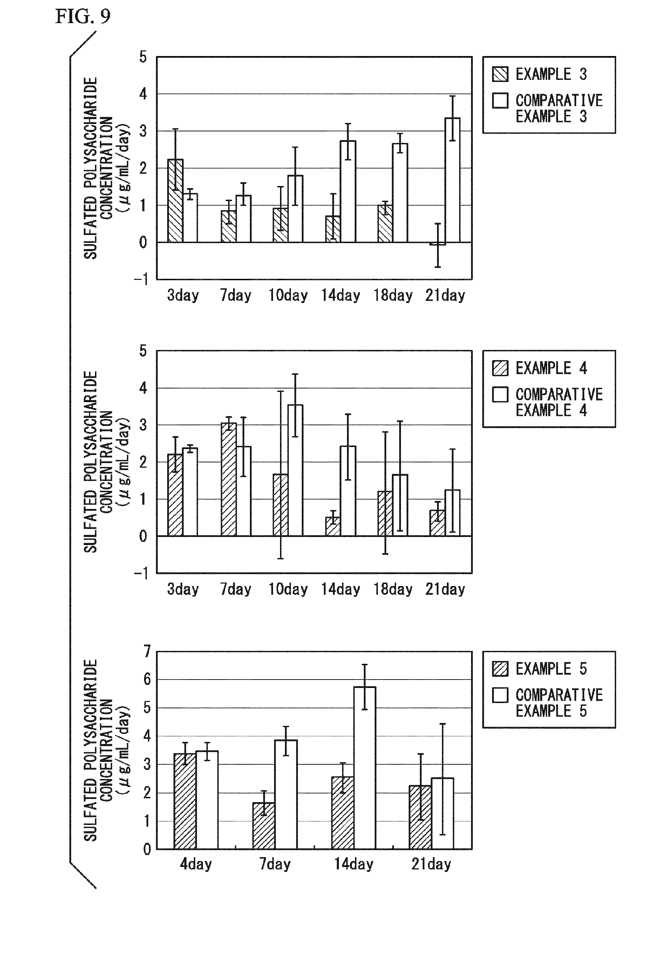

Sulfated polysaccharide present in the sampled culture supernatants was measured using a 1,9-dimethylmethylene blue (DMB) assay. The amount of sulfated polysaccharide secreted per day present in 1 mL of culture supernatant was determined from a calibration curve obtained from values obtained by measuring absorbance at 525 nm with a microplate reader (VersaMax, Molecular Devices Corp.) using a solution obtained by dissolving chondroitin sulfate A sodium salt in the media used in each of the examples and comparative examples (FIG. 9) .

### (4) Measurement of Amount of Sulfated Glycosaminoglycan (GAG) Produced

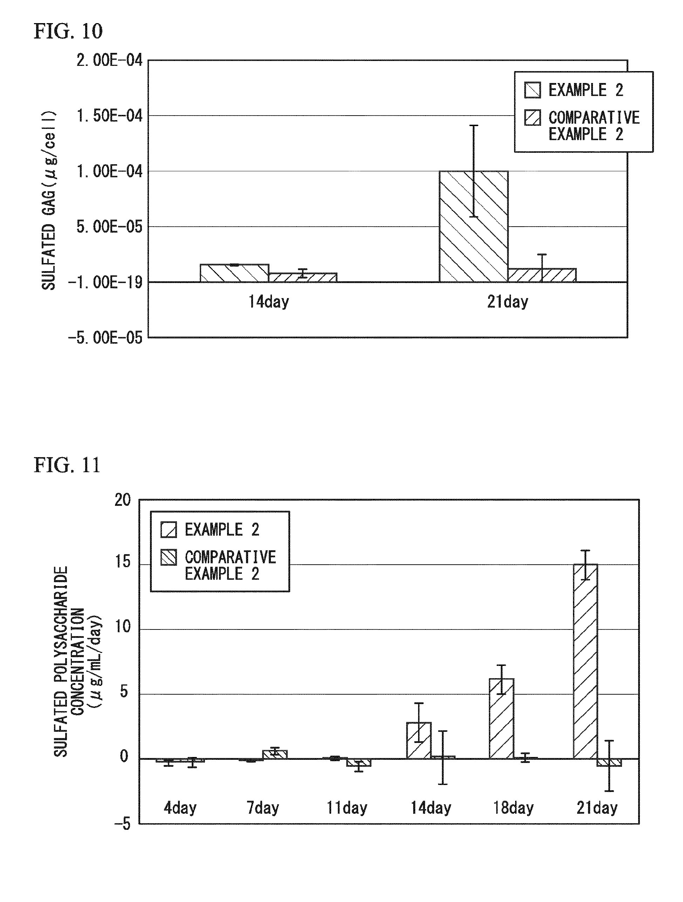

The sampled cells were lysed by treating for 3 hours at 60°C in a solution (containing 300 µg/mL of papain (Wako Pure Chemical Industries, Ltd.), 2 mM DTT (Nacalai Tesque Inc.) and 200 mg/L sodium dodecyl sulfate (Nacalai Tesque Inc.) followed by measuring using a 1,9-dimethylmethylene blue (DMB) assay. The value obtained from a calibration curve obtained from values measured at an absorbance at 525 nm obtained by measuring with a microplate reader (VersaMax, Molecular Devices Corp.) using the solution obtained by dissolving chondroitin sulfate A sodium salt in the aforementioned solution used when lysing the cells was divided by the number of cells to determined the amount of sulfated polysaccharide per cell (FIG. 10).

### (5) Measurement of Amount of Sulfated Polysaccharide

Sulfated polysaccharide present in the sampled culture supernatants was measured using a 1,9-dimethylmethylene blue (DMB) assay. The amount of sulfated polysaccharide secreted per day present in 1 mL of culture supernatant was determined from a calibration curve obtained from values obtained by measuring absorbance at 525 nm with a microplate reader (VersaMax, Molecular Devices Corp.) using a solution obtained by dissolving chondroitin sulfate A sodium in a medium obtained by adding 1% fetal bovine serum to DMEM (high glucose) (FIG. 11).

### (6) Measurement of Amount of Sulfated Polysaccharide Capable of Binding to rhBMP-2

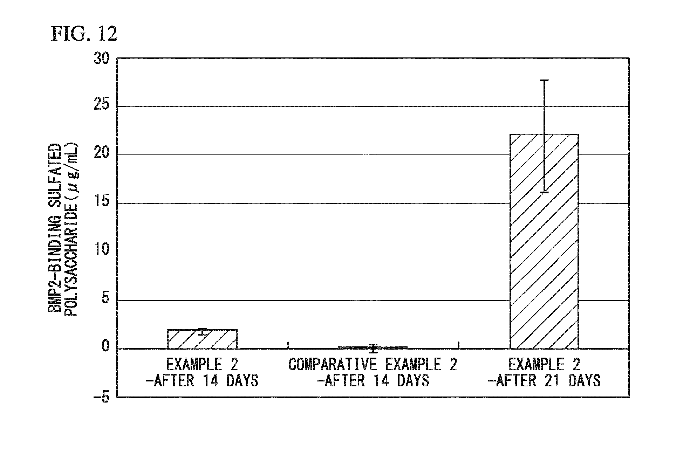

5 µL aliquots of heparin-Sepharose (GE Healthcare Corp.) were completely dispersed and washed twice with 50 µL of 10 mM phosphate buffer followed by treating for 10 minutes by respectively adding 100 µL of a solution obtained by dissolving rhBMP-2 in 10 mM phosphate buffer to 100 µg/mL, and 100 µL of 10 mM phosphate buffer. After removing the supernatant and washing twice with 200 µL of 10 mM phosphate buffer, the sampled culture supernatant (after 14 days and 21 days for Example 2 and after 14 days for Comparative Example 2) was added followed by treating for 30 minutes and recovering the supernatant. The amount of sulfated polysaccharide present in the recovered supernatant was measured using a 1,9-dimethylmethylene blue (DMB) assay. The amount of sulfated polysaccharide present in 1 mL of culture supernatant capable of binding to rhBMP-2 was determined by determining the amount of sulfated polysaccharide remaining in the supernatant from a calibration curve obtained from values obtained by measuring absorbance at 525 nm with a microplate reader (VersaMax, Molecular Devices Corp.) using a solution obtained by dissolving chondroitin sulfate A sodium salt in 10 mM phosphate buffer, and subtracting from the amount of sulfated polysaccharide present in the culture supernatant (FIG. 12).

### (7) Confirmation of Sulfated Polysaccharide Structure

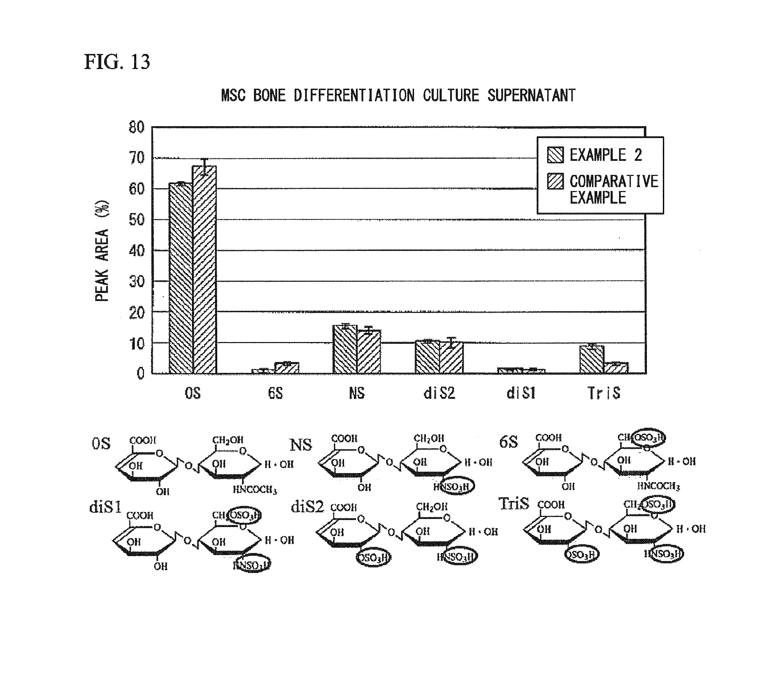

MilliQ was respectively added to 100 µL of medium obtained by adding 1% fetal bovine serum to 100 µL of the culture supernatant described in Example 2, and 100 µL of DMEM (high glucose); and bringing to a total volume of 500 µL, or to 500 µL of medium obtained by adding 1% fetal bovine serum to 500 µL of the culture supernatant described in Comparative Example 2, and 500 µL of DMEM (high glucose), followed by subjecting to centrifugal separation for 30 minutes at 14000G and 25°C using 0.5 mL of the Amicon Ultra™ 10K ultrafiltration membrane (Millipore Corp.). 480 µL of MilliQ were again added followed by again centrifuging for 40 minutes at 14000G and 25°C, and 10 µL aliquots were sampled from 25 µL aliquots of the recovered solutions, followed by the addition of 6 µL of digestion buffer (containing 10 mM sodium acetate trihydrate (Nacalai Tesque Inc.) and 10 mM potassium acetate hydrate (Nacalai Tesque Inc.), pH = 7.0), 5 µL of a solution obtained by dissolving heparinase (Seikagaku Corp.) in 0.1% aqueous BSA (Equitech-Bio Inc.) solution to a concentration of 0.5 U/mL, and 5 µL of a solution obtained by dissolving heparitinase (Seikagaku Corp.) in 0.1% aqueous BSA (Equitech-Bio Inc.) solution to a concentration of 0.5 U/mL, further adding MilliQ to a total volume of 30 µL and incubating for 1 hour at 37°C. Sugar chains were recovered by purifying 20 µL aliquots of these solutions and a 20 µL aliquot of a 100 µM mixed aqueous solution of six types of disaccharides contained in an unsaturated heparan/heparin-disaccharide kit (Seikagaku Corp.) using the BlotGlyco™ sugar chain purification kit (Sumitomo Bakelite Co., Ltd.). 5 µL aliquots of the recovered sugar chain solutions were analyzed by high-performance liquid chromatography ((LaChrom Elite, Hitachi High-Technologies Co., Ltd.). A normal phase chromatography column (TSK-GEL, Amide-80, Tosoh Corp.) was used for the column, and 50 mM aqueous ammonium formate solution (pH 4.4) and acetonitrile were used for the mobile phase. The mobile phase was given a concentration gradient so that the 50 mM aqueous ammonium formate solution (pH 4.4) changed from 20% to 41.7% over the course of 80 minutes, and sugar chain solutions were detected by fluorescence (excitation wavelength: 330 nm, fluorescence wavelength: 420 nm) by applying at a flow rate of 0.4 mL/min and at a column temperature of 30°C. The area of the medium was subtracted from the area of the culture supernatant for the peak corresponding to the elution time of a reference disaccharide peak from the resulting HPLC charts, and each of the peak areas was then divided by the total area of the six peaks and multiplied by 100 to calculate peak area (%) followed by a comparison of those values (FIG. 13).

### INDUSTRIAL APPLICABILITY

Use of the sulfated polysaccharide that binds with growth factor discovered by the inventors of the present invention makes it possible to promote differentiation of osteocytes both in vivo and in vitro. Thus, the composition of the present invention having this sulfated polysaccharide of the present invention as an active ingredient thereof can not only be used as a reagent for promoting cell proliferation and differentiation, but can also be used as a pharmaceutical for preventing or treating damaged cells, tissues or organs and diseases caused by abnormal cell proliferation and differentiation, and as a food or beverage such as a health food or beverage for improving cell proliferation and differentiation.

In addition, the degree of cell proliferation and differentiation in vivo or in vitro can be evaluated by targeting the sulfated polysaccharide that binds to growth factor discovered by the inventors of the present invention. A composition having this sulfated polysaccharide as an active ingredient thereof can be used as a diagnostic reagent for detecting cell proliferation and differentiation. The detection of this sulfated polysaccharide in a patient undergoing treatment for the purpose of regenerative medicine makes it possible to evaluate the effects of that treatment, thereby enabling the present invention to be used in the field of regenerative medicine as well.

## Claims

1. A sulfated polysaccharide secreted from cells during the course of cell proliferation and differentiation that binds to growth factor.

2. The sulfated polysaccharide according to claim 1, wherein the course of cell proliferation and differentiation is the course of proliferation and differentiation of osteocytes or chondrocytes.

3. The sulfated polysaccharide according to claim 1 or 2, wherein the growth factor is a molecule selected from the group consisting of BMP-2 and bFGF.

4. A method for evaluating cell proliferation and differentiation, comprising detecting the sulfated polysaccharide according to any of claims 1 to 3.

5. A composition for detecting cell proliferation and differentiation having as an active ingredient thereof a molecule that binds to the sulfated polysaccharide according to any of claims 1 to 3.

6. A composition for promoting cell proliferation and differentiation having as an active ingredient thereof the sulfated polysaccharide according to any of claims 1 to 3.
